(19) Europäisches Patentamt
European Patent Office
Office européen des brevets

(11) **EP 1 540 323 B1**

(12) **EUROPÄISCHE PATENTSCHRIFT**

(45) Veröffentlichungstag und Bekanntmachung des Hinweises auf die Patenterteilung:
**07.01.2009  Patentblatt 2009/02**

(21) Anmeldenummer: **03769206.8**

(22) Anmeldetag: **19.09.2003**

(51) Int Cl.:
***G01N 27/30*** (2006.01)

(86) Internationale Anmeldenummer:
**PCT/DE2003/003123**

(87) Internationale Veröffentlichungsnummer:
**WO 2004/029610 (08.04.2004 Gazette 2004/15)**

(54) **VERFAHREN ZUM HERSTELLEN EINER BIOSENSOR-SCHALTKREIS-ANORDNUNG MIT INTEGRIERTER REFERENZ-ELEKTRODE**

METHOD FOR PRODUCING BIOSENSOR SWITCHING CIRCUIT ARRANGEMENT COMPRISING AN INTEGRATED REFERENCE ELECTRODE

PROCÉDÉ DE FABRICATION D'UN CIRCUIT POUT BIO-CAPTEUR COMPRENANT UNE ÉLECTRODE DE RÉFÉRENCE INTÉGRÉE

(84) Benannte Vertragsstaaten:
**DE FR GB**

(30) Priorität: **19.09.2002  DE 10243569**

(43) Veröffentlichungstag der Anmeldung:
**15.06.2005  Patentblatt 2005/24**

(73) Patentinhaber: **SIEMENS AKTIENGESELLSCHAFT
80333 München (DE)**

(72) Erfinder: **PAULUS, Christian
82362 Weilheim (DE)**

(74) Vertreter: **Viering, Hans-Martin
Viering, Jentschura & Partner
Postfach 22 14 43
80504 München (DE)**

(56) Entgegenhaltungen:
**EP-A- 0 152 541          EP-A- 0 299 778
EP-A- 0 321 737          EP-A- 0 387 026
WO-A1-99/19507          US-A1- 2002 123 048**

• **DATABASE WPI Section Ch, Week 198152 Derwent Publications Ltd., London, GB; Class A85, AN 1981-95657D XP002269535 & JP 56 146218 A (MATSUSHITA ELEC IND CO LTD), 13. November 1981 (1981-11-13)**

## Beschreibung

Die Erfindung betrifft ein Verfahren zum Herstellen einer Biosensor-Schaltkreis-Anordnung.

In der chemischen und biochemischen Analysetechnik werden häufig elektrochemische Verfahren eingesetzt, um qualitative bzw. quantitative Informationen über eine Konzentration eines redoxaktiven Stoffs in einem Analyten zu gewinnen. Dabei werden häufig die Reduktions- bzw. Oxidationspotentiale der zu untersuchenden Stoffe sowie an Elektroden auftretende elektrische Stromwerte ermittelt und ausgewertet.

Das elektrochemische Potential einer metallischen Elektrode in einer Lösung kann unter Verwendung der Nernstschen Gleichung berechnet werden, die das Potential eines Redox-Systems gemäß dem folgenden Ausdruck beschreibt:

$$E = E_0 + (RT)/(nF) \ \log([Ox]/[Red]) \qquad (1)$$

Dabei ist E das elektrische Potential eines Redox-Systems, $E_0$ ein elektrisches Standard-Potential, R die Gaskonstante, T die absolute Temperatur, n die elektrochemische Wertigkeit, F die Faraday-Konstante, [Ox] die Konzentration der oxidierten Form und [Red] die Konzentration der reduzierten Form des Redox-Stoffes.

Aus Gleichung (1) ist ersichtlich, dass das elektrochemische Potential eines Redox-Systems von den Konzentrationen der Reaktionsteilnehmer abhängt. Aus diesem Grunde ist es erforderlich, ein Referenz-Potential bereitzustellen, das unabhängig von den Konzentrationsverhältnissen im Analyten einen Bezugspunkt für die elektrochemischen Potentiale bildet.

Ein derartiges Referenz-Potential wird mittels einer Referenz-Elektrode bereitgestellt.

Bei einer Referenz-Elektrode erster Art wird das elektrische Potential durch das elektrochemische Gleichgewicht zwischen dem Material der Elektrode und seinen Ionen in der Lösung bestimmt. Die Metallionen in der Lösung haben einen bestimmenden Einfluss auf das elektrische Potential. Ein Beispiel für eine Referenz-Elektrode erster Art ist die Wasserstoff-Elektrode. Anschaulich ermöglicht bei einer Elektrode erster Art eine elektrisch leitfähige Struktur den Ladungsaustausch zwischen zwei Medien.

Als Referenz-Elektrode zweiter Art ist eine Anordnung bekannt, in der die Konzentration der das elektrische Potential bestimmenden Ionen durch die Anwesenheit einer schwerlöslichen gleichionigen Verbindung, welche eine zweite feste Phase liefert, festgelegt ist. Eine Referenz-Elektrode zweiter Art ist ein Metall, das mit einer Schicht eines schwerlöslichen Salzes des Metalls bedeckt ist und in die Lösung eines leichtlöslichen Salzes eintaucht, das mit dem schwerlöslichen Salz ein gemeinsames Anion hat. Das Elektroden-Potential wird mittels der Aktivität der Anionen des leichtlöslichen Salzes festgelegt. Beispiele für Referenz-Elektroden zweiter Art ist die Kalomel-Elektrode oder die Silber/Silberchlorid-Elektrode.

Eine Ausführungsform einer Silber/Silberchlorid-Elektrode weist einen mit Silberchlorid-Salz überzogenen Silber-Draht auf, der in eine Chloridionen-haltige Lösung eintaucht. Die Konzentration der Silberionen ist aufgrund der Anwesenheit der Chloridionen in der Lösung sehr klein und durch das Gleichgewicht

$$Ag^+ + Cl^- \leftrightarrow AgCl \qquad (2)$$

festgelegt.

Gemäß dem Stand der Technik werden in elektrochemischen Analyseverfahren makroskopische Referenz-Elektroden, insbesondere eine Silber/Silberchlorid-Elektrode, verwendet, die mit dem Analyten in Wirkkontakt gebracht werden. Typische Abmessungen derartiger Elektroden reichen von mehreren Zentimetern bis zu wenigen Millimetern.

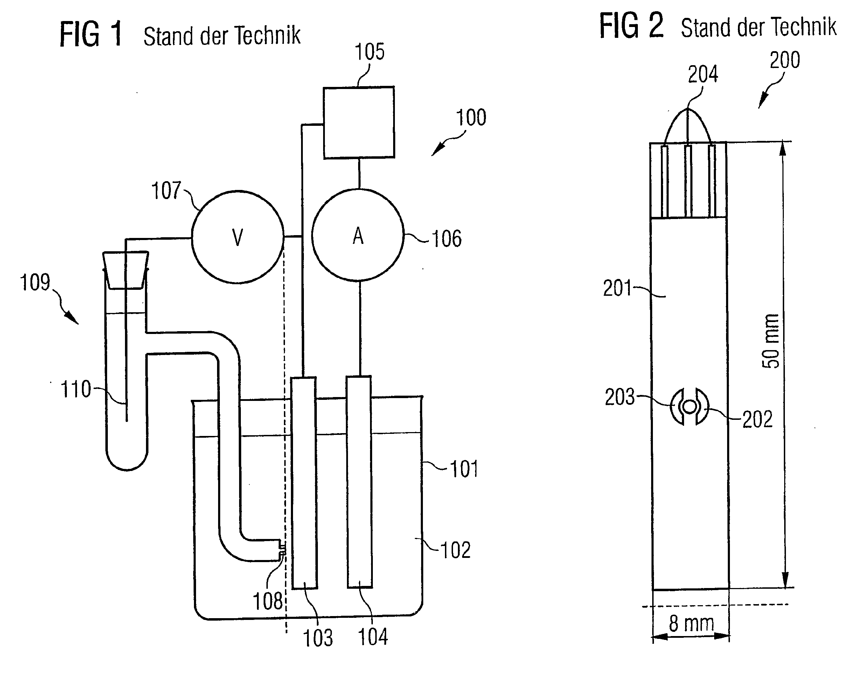

In **Fig.1** ist eine elektrochemische Experimentier-Anordnung 100 gemäß dem Stand der Technik gezeigt. In einem Behälter 101 ist ein Analyt 102 eingefüllt, der mittels Arbeits-Elektroden 103, 104 elektrochemisch untersucht wird. Die erste Arbeits-Elektrode 103 ist mit einer Stromversorgung 105 gekoppelt, wohingegen die zweite Arbeits-Elektrode 104 mit einem Amperemeter 106 gekoppelt ist. Ferner ist zwischen die erste Arbeits-Elektrode 103 und einem mit Silberchlorid-Material überzogenen Silberdraht 110 einer Silber/Silberchlorid-Elektroden-Anordnung 109 ein Voltmeter 107 geschaltet. Mittels einer dünnen Kapillare 108 ist der Analyt 102 mit der Silber/Silberchlorid-Elektroden-Anordnung 109 in Wirkkontakt gebracht. Aufgrund der Funktionalität der Silber/Silberchlorid-Elektroden-Anordnung 109 ist dem Analyt 102 der elektrochemischen Experimentier-Anordnung 100 ein elektrisches Referenz-Potential bereitgestellt. Anschaulich ist die Experimentier-Anordnung zum Untersuchen des Analyten 102 eingerichtet, mittels der Silber/Silberchlorid-Elektroden-Anordnung 109 wird für die Untersuchung ein Referenz-Potential bereitgestellt.

Allerdings weist die in Fig.1 gezeigte Silber/Silberchlorid-Referenz-Elektrode 109 den Nachteil auf, dass ein erhebliches Mindestvolumen des Analyten 102 erforderlich ist, um die makroskopische Referenz-Elektrode zuverlässig mit dem Analyten 102 in Wirkkontakt zu bringen. Ferner ist der mechanische Aufbau der Silber/Silberchlorid-Elektroden-Anordnung 109 aufwändig, weshalb derartige Referenz-Elektroden nur in Laborsystemen zur Anwendung kommen. Insbesondere für biosensorische Anwendungen ist die Silber/Silberchlorid-Elektroden-Anordnung 109 ungeeignet, da in der Biologie in der Regel nur geringe Probenvolumina zur Verfügung stehen.

Im Weiteren wird bezugnehmend auf Fig.2 eine aus [1] bekannte Sensor-Anordnung 200 für elektrochemische Messungen beschrieben, die eine Referenz-Elektrode aufweist.

Die Sensor-Anordnung 200 weist eine Dimension von 8mm mal 50mm auf und hat eine Arbeits-Elektrode 201, eine GegenElektrode 202 und eine Referenz-Elektrode 203. Ferner sind Kontaktierungen 204 bereitgestellt, mittels welcher die Sensor-Anordnung 200 mit einem Steuer-Schaltkreis koppelbar ist. Die Sensor-Anordnung 200 weist eine in der Elektrochemie übliche Drei-Elektroden-Anordnung mit den Elektroden 201, 202, 203 auf und ist unter Verwendung eines Siebdruck-Verfahrens hergestellt. Die Arbeits-Elektrode 201 ist aus Kohlenstoff, wohingegen die Gegen-Elektrode 202 und die Referenz-Elektrode 203 jeweils als Silber/Silberchlorid-Elektrode ausgebildet ist.

Um die Konzentration eines Analyten zu ermitteln, wird der Analyt auf die Elektroden 201 bis 203 pipettiert, wobei ein Volumen von mindestens 15µl erforderlich ist. Derartige Probenvolumina stehen insbesondere bei biologischen Untersuchungen bei ausreichend starker Probenkonzentration häufig nicht zur Verfügung.

In miniaturisierten Systemen wird häufig ein chlorierter Silberdraht als Referenz-Elektrode eingesetzt, da ein solcher in relativ geringer Abmessung ausgebildet werden kann. Hierbei wird ein Silber-Draht oder ein versilberter Platindraht oberflächlich chloriert und anschließend mit einem Analyten in Wirkkontakt gebracht. Wie beispielsweise in [2] beschrieben, ist dadurch eine Referenz-Elektrode mit einer Dimension im Millimeter-Bereich ausbildbar, eine noch stärkere Miniaturisierung ist aber nicht möglich. Daher eignen sind die aus [2] bekannten Dri-Ref™ Referenz-Elektroden vorwiegend für den Laborbetrieb, wohingegen sie für kommerzielle Anwendungen ungeeignet sind.

Insbesondere in der DNA-Sensorik, zum Beispiel bei Redox-Recycling-Sensoren, ist die Kenntnis des elektrischen Potentials eines Elektrolyten wichtig. Daher ist eine Referenz-Elektrode bei einer DNA-Sensor-Anordnung erforderlich, wenn elektrochemische Analyseverfahren eingesetzt werden. Bei einem DNA-Sensor beträgt der Durchmesser des biologisch-aktivierten Bereichs einer Sensor-Anordnung häufig zwischen 100µm und 200µm, wobei typischerweise zehn bis hundert DNA-Sensoren pro $mm^2$ Substrat-Oberfläche vorgesehen sind.

Diese Dimensionsangaben resultieren aus der Dimension, die bei Aufbringen geeigneter DNA-Fängermoleküle (z.B. DNA-Halbstränge) auf die Sensor-Oberfläche unter Verwendung einer Piezo- oder Nadeltechnik erreichbar sind. Bei der anschaulich als Tintenstrahl-Technologie bekannten Piezo-Technologie wird das biologische Agens mittels eines Druckkopfes, der jenen ähnelt, die bei Büro-Tintenstrahldruckern verwendet werden, kontaktlos in kleinen Tröpfchen auf die Sensor-Oberfläche aufgebracht. Bei der anschaulich als Stempeltechnologie bezeichneten Nadel-Technologie arbeitet die Druckvorrichtung vergleichbar einem Nadeldrucker. Die Drucknadel wird in einem Ring, der das zu druckende biologische Agens enthält, mit der Flüssigkeit benetzt und anschließend auf die Sensor-Oberfläche aufgedrückt. Grundlagen dieser Technologien sind beispielsweise in [3] beschrieben. In [4] wird eine Übersicht über gängige Geräte und Verfahren zum Herstellen von Mikroarrays gegeben.

[5] offenbart ein Verfahren zur Herstellung einer Kombination eines Drucksensors und eines elektrischen Sensors.

[6] offenbart einen elektrochemischen Sensor auf einem Substrat mit einer Edelmetallelektrode.

[7] offenbart eine elektrochemische Metallanalysevorrichtung mit einer gedruckten Elektrode.

[8] offenbart einen planaren Gassensor, insbesondere einen $pCO_2$- oder $pO_2$-Sensor.

[9] offenbart einen MOS-Transistor mit einer Gate-Elektrode, die elektrosch leitfähig mit einem freigelegten Kontaktbereich gekoppelt ist.

WO 99/19057 offenbart ein Verfahren zur Herstellung einer Biosensor-Schaltkreis-Anordnung, wobei Referenzelectrode und biologische Molükule auf ein Substrat gedruckt werden.

EP 0 299 778 A2 offenbart ein Verfahren zur Herstellung eines elektrochemischen Mikrosensors, enthaltend eine Referenzelektrode und Elektrodenstrukturen mit aufgedruckten biologisch aktiven Molekülen.

Der Erfindung liegt das Problem zugrunde, eine kostengünstige und mit vertretbarem Aufwand herstellbare Referenz-Elektrode bereitzustellen, die sich auch für Anwendungen der Biosensorik eignet.

Das Problem wird durch ein Verfahren zum Herstellen einer Biosensor-Schaltkreis-Anordnung mit den Merkmalen gemäß den unabhängigen Patentansprüchen gelöst.

Anschaulich ist erfindungsgemäß eine miniaturisierte, monolithisch integrierte Referenz-Elektrode zweiter Art ausgebildet, wobei der integrierte Schaltkreis der Schaltkreis-Anordnung mit der Referenz-Elektrode zweiter Art elektrisch gekoppelt ist, so dass die Referenz-Elektrode auf ein elektrisches Referenz-Potential bringbar ist. Die erfindungsgemäße elektrochemische Referenz-Elektrode ist insbesondere auf die Bedürfnisse hochintegrierter elektrochemischer Analysesysteme zugeschnitten. Die Referenz-Elektrode der Erfindung kann vorteilhaft auf der Oberfläche einer als Sensor-Anordnung ausgestalteten Schaltkreis-Anordnung verwendet werden, und kann insbesondere mit vergleichbaren Abmessungen wie integrierte Bauelemente (z.B. ein integriertes Biosensor-Element) ausgebildet werden. Der Aufwand zum Herstellen der erfindungsgemäßen Schaltkreis-Anordnung ist gering, so dass sich die Referenz-Elektrode insbesondere für die Massenproduktion eignet, z.B. von Biosensor-Anordnungen. Mittels der Schaltkreis-Anordnung ist mit geringem Aufwand und hoher Genauigkeit ein Referenz-Potential bereitstellbar. Dies ist insbesondere im Hinblick auf die Technologie eines vollelektronischen monolithischen Biosensors vorteilhaft, da für solche Anwendungen aus dem Stand der Technik keine befriedigenden Lösungen zum Bereitstellen eines Referenz-Potentials bekannt sind. Die miniaturisierte Referenz-Elektrode (die insbesondere als Silber/Silberchlorid-Referenz-Elektrode ausgestaltet sein kann) kann mit geringem Aufwand (beispielsweise unter Verwendung einfacher Druck- oder elektrochemischer Verfahren) hergestellt werden. Zur Herstellung der Elektrode sind aufwendige und daher kostenintensive Prozesstechniken der Halbleiterindustrie entbehrlich.

Bevorzugte Weiterbildungen der Erfindung ergeben sich aus den abhängigen Ansprüchen.

Vorzugsweise kann der integrierte Schaltkreis derart eingerichtet sein, dass ihm von der Referenz-Elektrode ein für das elektrische Potential in einem Umgebungsbereich der Referenz-Elektrode charakteristisches Signal bereitstellbar ist.

Mit anderen Worten kann die Referenz-Elektrode das elektrische Potential in ihrem Umgebungsbereich erfassen (der Umgebungsbereich ist z.B. ein Elektrolyt, in den die Referenz-Elektrode eintaucht) und dem Schaltkreis ein entsprechendes Signal bereitstellen. Der Schaltkreis kann ferner derart eingerichtet sein, dass er einer zum Beispiel in den Elektrolyten eingetauchten Gegenelektrode basierend auf dem erfassten Potential ein Steuer-Signal bereitstellt, das derart gewählt ist, dass das elektrische Potential des Elektrolyten mittels der Gegenelektrode beeinflusst wird (z.B. das Potential auf den Wert eines Referenz-Potentials eingestellt wird).

Die Kopplung zwischen dem integrierten Schaltkreis und dem Kern der Referenz-Elektrode kann als direkte Kopplung eines elektrisch leitfähigen Kopplungsmittels des integrierten Schaltkreises mit dem metallischen Kern der Referenz-Elektrode realisiert sein.

Alternativ kann die Schaltkreis-Anordnung eine elektrisch leitfähige Kopplungsstruktur aufweisen, mittels welcher der integrierte Schaltkreis mit dem metallischen Kern elektrisch gekoppelt ist. Die Kopplungsstruktur ist vorzugsweise aus einem chemisch inerten Material wie Gold oder Platin hergestellt.

Das Metall des Kerns der Referenz-Elektrode ist Silber, und das Salz des Metalls ist vorzugsweise Silberchlorid. Dann ist die Referenz-Elektrode als Silber/Silberchlorid-Referenz-Elektrode ausgestaltet. Alternativ ist aber auch jede andere Metall-Metallsalz-Kombination geeignet, sofern das Salz ausreichend schwerlöslich ist (beispielsweise Silber/Silberjodid).

Das Substrat kann zum Beispiel ein Halbleiter-Material (insbesondere Silizium), Glas, Kunststoff und/oder Keramik aufweisen. Vorzugsweise ist das Substrat ein Silizium-Wafer.

Die Schaltkreis-Anordnung ist als Sensor-Anordnung eingerichtet. In einem solchen Fall kann die Referenz-Elektrode der Schaltkreis-Anordnung dazu dienen, ein elektrisches Potential der Sensor-Anordnung konstant zu halten.

Die Schaltkreis-Anordnung ist eingerichtet als Biosensor-Anordnung. Beispielsweise kann die Schaltkreis-Anordnung eine Biosensor-Anordnung zur DNA-Analyse unter Verwendung eines elektrochemischen Prozesses (z.B. Redox-Recycling) sein. Das für den Betrieb der auf einem Halbleiter-Chip als Substrat enthaltenen Potentiostaten erforderliche Referenz-Potential kann unter Verwendung der erfindungsgemäßen Referenz-Elektrode bereitgestellt werden.

Im Weiteren wird das erfindungsgemäße Verfahren zum Herstellen einer Schaltkreis-Anordnung näher beschrieben. Ausgestaltungen der Schaltkreis-Anordnungen gelten auch für Verfahren zum Herstellen einer Schaltkreis-Anordnung.

Eine elektrisch leitfähige Kopplungsstruktur kann derart ausgebildet werden, dass mit dieser der integrierte Schaltkreis mit dem Kern elektrisch gekoppelt wird.

Vorzugsweise wird als metallisches Material des Kerns Silber verwendet. Als Salz des Metalls zum Ausbilden der Umhüllung des metallischen Kerns wird vorzugsweise Silberchlorid verwendet.

Der Kern wird mittels Bedruckens des Substrats und/oder der Kopplungsstruktur mit Silber-Material ausgebildet.

Gemäß einer weiteren Alternative kann der Kern ausgebildet werden, indem das Substrat und/oder die Kopplungsstruktur mit Silbersalz-Material unter Verwendung einer an sich bekannten Drucktechnik bedruckt wird und das Silbersalz-Material chemisch zu Silber reduziert wird.

Bei jeder der beschriebenen Alternativen kann der Kern zumindest teilweise von der Hülle umgeben werden, indem der Kern aus Silber unter Verwendung eines elektrochemischen Verfahrens oder eines chemischen Verfahrens chloriert wird.

Mit dem erfindungsgemäßen Verfahren ist es ermöglicht, eine Referenz-Elektrode auf und/oder in dem Substrat zu integrieren. Dadurch ist ein nachträgliches, aufwendiges Anbringen der Referenz-Elektrode entbehrlich. Als Substrat eignen sich alle Materialien, auf denen elektrisch leitfähige Strukturen als Komponente der Referenz-Elektrode aufgebracht werden können, wie beispielsweise Glas, Keramik, Kunststoff. Bevorzugt verwendet wird ein Halbleiter-Substrat, mit dem es ermöglicht ist, die zum Betrieb einer Sensor-Anordnung bzw. zum Bereitstellen einer Konstantspannung erforderliche elektrische Schaltung zu integrieren, vorzugsweise in CMOS-Technologie.

Bevor die eigentliche Referenz-Elektrode auf dem Substrat ausgebildet wird, wird vorzugsweise eine elektrisch leitfähige Kopplungsstruktur (anschaulich auch Grund-Elektrode genannt) auf der Chip-Oberfläche aufgebracht. Mittels der Grund-Elektrode kann ein elektrischer Kontakt zwischen der auszubildenden Referenz-Elektrode und dem in dem Substrat integrierten Schaltkreis realisiert werden. Vorzugsweise ist die Grund-Elektrode aus einem chemisch inerten Material wie Gold oder Platin gefertigt.

Insbesondere ist es bei der Ausgestaltung der Schaltkreis-Anordnung als Biosensor-Anordnung vorteilhaft, die Referenz-Elektrode der Erfindung in dem gleichen Arbeitsgang und unter Verwendung der gleichen oder einer ähnlichen Vorrichtung auszubilden, mit der die Oberfläche des Substrats biologisch aktiviert wird. Insbesondere ist eine druckbare Referenz-Elektrode vorteilhaft.

Ausführungsbeispiele der Erfindung sind in den Figuren dargestellt und werden im Weiteren näher erläutert.

Es zeigen:

Figur 1          eine elektrochemische Experimentier-Anordnung gemäß dem Stand der Technik,

Figur 2          eine Sensor-Anordnung gemäß dem Stand der Technik,

Figuren 3A bis 3C     Schichtenfolgen zu unterschiedlichen Zeitpunkten während eines Verfahrens zum Herstellen einer Schaltkreis-Anordnung gemäß einem bevorzugten Ausführungsbeispiel der Erfindung,

Figuren 4A bis 4C     eine als Redox-Recycling-Biosensor- Anordnung ausgestaltete Schaltkreis-Anordnung gemäß einem bevorzugten Ausführungsbeispiel der Erfindung.

Um eine erfindungsgemäße Schaltkreis-Anordnung herzustellen, die eine Referenz-Elektrode aus einem Silber-Kern und einer Silberchlorid-Umhüllung aufweist, wird zunächst der Kern aus Silber auf dem Substrat ausgebildet. Der Silber-Kern wird anschließend zumindest teilweise von einer Hülle aus schwerlöslichem Silberchlorid umgeben.

Zunächst werden Ausführungsbeispiele beschrieben, wie metallisches Silber auf ein Substrat abgeschieden werden kann, um einen Silber-Kern auf einem Substrat auszubilden.

Der Silber-Kern wird mittels Bedruckens des Substrats (bzw. mittels Bedruckens einer zuvor ausgebildeten Kopplungsstruktur auf dem Substrat zum Herstellen einer elektrischen Kopplung zwischen dem integrierten Schaltkreis des Substrats und dem Silber-Kern) mit Silber-Material ausgebildet werden.

Hierfür wird beispielsweise eine Lösung von Silber-Partikeln in einem Nitrocellulose-Lack auf das Substrat oder auf die Kopplungsstruktur (Grund-Elektrode) aufgedruckt, wobei das oben beschriebene Stempel-Verfahren oder das oben beschriebene Tintenstrahl-Verfahren verwendet werden kann. Nach einem Trocknungsvorgang bildet sich ein elektrisch leitfähiger Überzug auf dem Substrat bzw. auf der Grund-Elektrode. Dieser Überzug kann direkt als Metall-Kern der Referenz-Elektrode dienen oder alternativ zusätzlich galvanisch verstärkt werden. Zum galvanischen Verstärken wird die erhaltene Schichtenfolge in eine Lösung eines SilberSalzes eingetaucht, und es wird eine elektrische Spannung an den metallischen Kern angelegt. Diese Spannung kann beispielsweise unter Verwendung der Funktionalität des bereits in dem Substrat ausgebildeten integrierten Schaltkreises angelegt werden. Gemäß dieser Vorgehensweise kann die Form der gebildeten Referenz-Elektrode flexibel mittels Justieren des Druckvorgangs eingestellt werden. Dieses Verfahren weist den Vorteil auf, dass zum Aufbringen des Silber-Kerns die gleiche Drucktechnologie verwendet werden kann, wie sie auch zum biologischen Aktivieren eines Sensorfeldes verwendet wird. Der Silber-Kern der Referenz-Elektrode wird im gleichen Arbeitsschritt aufgebracht, in dem auch die Sensorfelder einer als Biosensor- Anordnung ausgestalteten Schaltkreis-Anordnung mit geeigneten Fängermolekülen (beispielsweise DNA-Halbsträngen) versehen werden. Optional kann ein spezieller Reinigungsschritt des Druckkopfes erforderlich sein, wenn das Aufbringen von Silber-Material auf das Substrat bzw. auf die Grund-Elektrode unter Verwendung einer organischen Lösung von Silber-Partikeln realisiert ist, wohingegen bei biologischen Agenzien häufig eine wässrige Lösung der Biomoleküle verwendet wird. Gemäß einem weiteren alternativen Verfahren zum Ausbilden eines Silber-Kerns auf dem Substrat (bzw. auf der Grund-Elektrode) wird der Silber-Kern ausgebildet, indem das Substrat und/oder die Grund-Elektrode mit Silbersalz-Material bedruckt wird und das Silbersalz-Material chemisch zu Silber reduziert wird. Dieses Verfahren kann anschaulich als Abscheiden eines Silber-Spiegels bezeichnet werden. Hierfür wird auf die Grund-Elektrode eine Silbersalz-Lösung aufgedruckt, beispielsweise eine ammoniakalische Silbernitrat-Lösung, und unmittelbar danach ein Reduktionsmittel, beispielsweise eine Hydrazin-Lösung ($H_2N-NH_2$), zugegeben. Dadurch werden die in der Salzlösung enthaltenen Silberionen chemisch reduziert, mit der Folge, dass sich auf der von der Salzlösung benetzten Oberfläche des Chips elementares Silber als Kern der erfindungsgemäßen Referenz-Elektrode abscheidet. Dieses Verfahren hat den wesentlichen Vorteil, dass ein Galvanisieren entbehrlich ist, um eine geschlossene Silber-Schicht auf der Grund-Elektrode abzuscheiden. Die Qualität der Silber-Schicht hängt dabei stark von den chemischen Bedingungen, unter denen die Reduktionsreaktion abläuft, sowie von der Beschaffenheit der Oberfläche ab, auf welcher der Silber-Spiegel abgeschieden wird. Die geometrische Form einer derartig ausgebildeten Referenz-Elektrode ist durch die geometrische Form der aufgedruckten Silbersalz-Lösung bestimmt und kann von der Form der Grund-Elektrode unabhängig ausgebildet werden.

Die Silbersalz-Lösung einerseits und das Reduktionsmittel andererseits können auch in bereits vermischter Form aufgedruckt werden. Mischt man eine ammoniakalische Silber-Nitratlösung ($AgNO_3$ mit $NH_3$) mit Glukoselösung und druckt dieses Gemisch auf die Grund-Elektrode auf, so scheidet sich ein Silber-Spiegel auf der bedruckten Oberfläche ab. Diese Reaktion wird wesentlich beschleunigt, indem die Mischung nach dem Aufdrucken erwärmt wird oder auf eine bereits erwärmte Oberfläche aufgedruckt wird. Werden Silbersalz-Lösung und Reduktionsmittel nacheinander aufgedruckt, so kann mittels geeigneter Stoff-Auswahl, insbesondere des Reduktionsmittels, und mittels Einstellens der Konzentrationen das Abscheiden des Silber-Spiegels erheblich beschleunigt werden, so dass auch ein Erhitzen des Gemisches zum Versilbern entbehrlich ist.

Auch dieses Verfahren weist den großen Vorteil auf, dass zum Aufbringen der Silber-Elektrode die gleiche Drucktechnologie verwendet werden kann, wie sie zum biologischen Aktivieren der Sensor-Felder (z.B Aufbringen von DNA-Halbsträngen) verwendet wird. Im Idealfall kann die Silber-Elektrode sogar im gleichen Arbeitsschritt aufgebracht werden wie die biologischen Moleküle. Ferner ist vorteilhaft, dass es sich ausschließlich um eine wässrige Lösung der eingesetzten Stoffe handelt, so dass zum Reinigen des Druckkopfes keine besonderen Maßnahmen erforderlich sind, wie dies beispielsweise bei der Verwendung organischer Lösungsmittel der Fall ist.

Im Weiteren werden Ausführungsbeispiele für Verfahren beschrieben, wie der gemäß irgendeinem der oben beschriebenen Verfahren ausgebildete Silber-Kern zumindest teilweise von einer Silberchlorid-Hülle umgeben wird.

Dies kann realisiert werden, indem der Silber-Kern unter Verwendung eines elektrochemischen Verfahrens chloriert wird.

Beim elektrochemischen Chlorieren wird der Silber-Kern in eine Chloridionen-haltige Lösung getaucht, beispielsweise Natriumchlorid (NaCl) oder Kaliumchlorid (KCl). Da Silber ein relativ edles Metall ist, ist vorzugsweise eine elektrische Spannung an die Elektroden anzulegen, so dass die Reaktion

$$Ag + Cl^- \rightarrow AgCl + e^- \qquad (3)$$

ausreichend schnell abläuft. Das an der Gegen-Elektrode entstehende Natrium-Material ($Na^+ + e^- \rightarrow Na$) bzw. Kalium-Material ($K^+ + e^- \rightarrow K$) reagiert in der wässrigen Lösung zu Natronlauge (NaOH) bzw. zu Kalilauge (KOH), wobei Wasserstoff frei wird:

$$2Na + 2H_2O \rightarrow 2NaOH + H_2 \qquad (4)$$

bzw.

$$2K + 2H_2O \rightarrow 2KOH + H_2 \qquad (5)$$

Wie bereits für den Fall der Silber-Galvanisierung der Grund-Elektrode beschrieben, erfolgt das Chlorieren des Silber-Kerns bevorzugt, bevor die Chips aus einem Wafer ausgesägt werden. Mittels geeigneter elektrisch leitfähiger Kopplungsmittel zwischen dem Chips auf dem Wafer sind zunächst alle Grund-Elektroden (oder ein Teil der Grund-Elektroden) miteinander elektrisch gekoppelt. Mittels Eintauchens des Wafers in eine geeignete Chloridionen-haltige Salzlösung und mittels Anlegen einer geeigneten Spannung an die so gebildete Elektroden-Anordnung werden alle Silber-Elektroden aller Chips des Wafers in einem gemeinsamen Verfahrensschritt chloriert. Beim anschließenden Aussägen der Chips werden automatisch die elektrischen Kopplungen zwischen den Grund-Elektroden durchtrennt. Auch das galvanische Chlorieren der Silber-Elektrode kann auf dem einzelnen Chip realisiert werden, wenn mehrere Elektroden in ausreichender Nähe zueinander auf der Chip-Elektrode vorliegen. Auf die Silber-Kerne wird eine chloridhaltige Salzlösung (beispielsweise NaCl oder KCl-Lösung) pipettiert, an die Silber-Kerne wird (vorzugsweise unter Verwendung der damit gekoppelten integrierten Schaltkreise) ein positives elektrisches Potential angelegt, und eine der anderen Elektroden, die ebenfalls mit der Salzlösung in Wirkkontakt ist, wird als Gegen-Elektrode verwendet.

Alternativ kann der Silber-Kern zumindest teilweise von der Hülle umgeben werden, indem der Silber-Kern unter Verwendung eines chemischen Verfahrens chloriert wird.

Das chemische Chlorieren kann realisiert werden, indem der Silber-Kern beispielsweise in eine saure Eisen-(III) chloridlösung eingetaucht wird, wodurch eine Chlorierungs-Reaktion des Silbers ausgelöst wird. Das Redoxsystem Ag/Ag+ weist ein Normalpotential von 0.799V auf, und das Redoxsystem $Fe^{3+}/Fe^{2+}$ weist ein Normpotential von 0.771V (pH=1) auf, so dass eine selbständige Chlorierung nur dann abläuft, wenn beispielsweise durch eine Koordination mit Komplexliganden das Silber-Potential abgesenkt wird. So weist das Redoxsystem Ag/AgCl ein Normpotential von nur 0.222V auf. Dies bedeutet, dass der Silber-Kern mittels einfachen Ausdruckens einer sauren Eisenchlorid-Lösung ($FeCl_3$) chloriert werden kann. Die Redox-Gleichungen für diese Reaktion lauten:

$$Ag \rightarrow Ag^+ + e^- \; (E_0=0.222V \text{ in Anwesenheit von } Cl^-) \qquad (6)$$

$$Fe^{3+} + e^- \rightarrow Fe^{2+} \quad (E_0=0.771 \text{ Volt bei pH}=1) \qquad (7)$$

Auch dieses Verfahren weist den Vorteil auf, dass zum Chlorieren des Silber-Kerns vorzugsweise die gleiche Drucktechnologie genutzt wird, wie sie zum biologischen Aktivieren von Sensor-Feldern verwendet wird. Die Eisen (III)-chlorid-Lösung kann im gleichen Arbeitsschritt aufgebracht werden wie die biologisch aktiven Fängermoleküle. Ferner ist es vorteilhaft, dass ausschließlich wässrige Lösungen der eingesetzten Stoffe verwendet werden. Dadurch ist die Reinigung des Druckkopfes ohne besondere Maßnahmen möglich, wie dies beispielsweise bei der Verwendung organischer Lösungsmittel der Fall ist.

Als weiteres Chlorierungs-Verfahren sei ferner das direkte Chlorieren der Silber-Elektrode genannt, bei der Substanzen verwendet werden, die elementares Chlor enthalten, wie beispielsweise Königswasser ($HNO_3+3HCl$). Aufgrund der hohen Reaktionsfreudigkeit derartiger Stoffe sind insbesondere beim Drucken besondere Maßnahmen nötig.

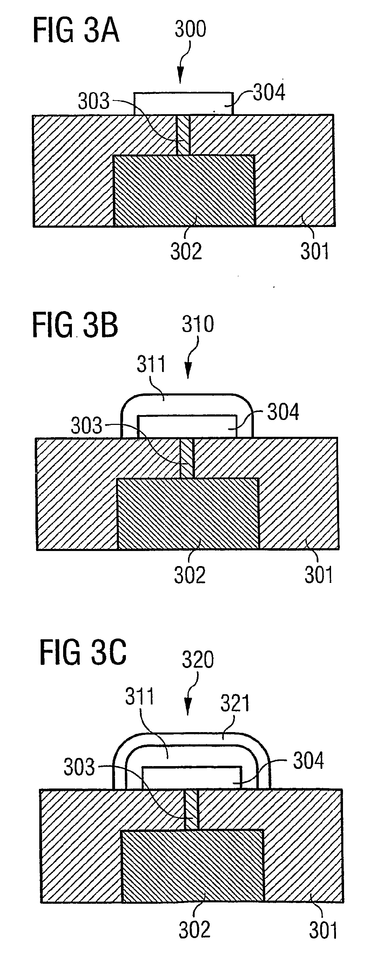

Im Weiteren wird bezugnehmend auf Fig.3A bis Fig.3C ein bevorzugtes Ausführungsbeispiel des erfindungsgemäßen Verfahrens zum Herstellen einer Schaltkreis-Anordnung beschrieben.

Um die in **Fig.3A** gezeigte Schichtenfolge 300 zu erhalten, wird in einem Silizium-Substrat 301 zunächst ein integrierter Schaltkreis 302 ausgebildet. Unter Verwendung eines geeigneten Ätzverfahrens wird ein Durchgangsloch in die Oberfläche des Silizium-Substrats 301 geätzt und dieses Kontaktloch mit einem Wolfram-Kontaktierungselement 303 gefüllt. Dann wird die Oberfläche der so erhaltenen Schichtenfolge mit einer durchgehenden Gold-Schicht bedeckt und unter Verwendung eines Lithographie- und eines Ätzverfahrens die Gold-Schicht derart zurückgeätzt, dass dadurch die Gold-Grund-Elektrode 304 zurückbleibt. Wie in Fig.3A gezeigt, ist die Gold-Grund-Elektrode 304 über das elektrisch leitfähige Wolfram-Kontaktierungselement 303 mit dem integrierten Schaltkreis 302 gekoppelt.

Um die in **Fig.3B** gezeigte Schichtenfolge 310 zu erhalten, wird Silber-Material 311 als Kern einer Referenz-Elektrode auf der Gold-Grund-Elektrode 304 abgeschieden. Gemäß dem beschriebenen Ausführungsbeispiel wird dies realisiert, indem die Gold-Grund-Elektrode 304 mit einer Silbersalz-Lösung bedruckt wird. Anschließend wird unter Verwendung eines Reduktionsmittels das Silbersalz-Material chemisch zu elementarem Silber-Material reduziert, wodurch anschaulich ein Silber-Spiegel 311 ausgebildet wird. Gemäß dem beschriebenen Ausführungsbeispiel wird als Silbersalz-Lösung eine ammoniakalische Silbernitrat-Lösung ($AgNO_3$ mit $NH_3$) und als Reduktionsmittel wird eine Glukose-Lösung verwendet.

Um die in **Fig.3C** gezeigte Schaltkreis-Anordnung 320 zu erhalten, wird der Kern aus Silber-Material 311 unter Verwendung eines chemischen Verfahrens chloriert, wodurch die Silberchlorid-Umhüllung 321 ausgebildet wird. Die Chlorierung erfolgt mittels Zugabe von Eisen(III)chlorid, wodurch eine dünne, poröse Silberchlorid-Schicht 321 ausgebildet wird. Der Silber-Kern 311 und die Silberchlorid-Umhüllung 321 bilden eine integrierte Referenz-Elektrode, an die mittels der elektrisch leitfähigen Grund-Elektrode 304 und mittels des elektrisch leitfähigen Wolfram-Kontaktierungselements 303 eine elektrische Kopplung mit dem integrierten Schaltkreis 302 realisiert ist. Der integrierte Schaltkreis 302 ist derart eingerichtet, dass ihm von der Referenz-Elektrode ein für das elektrische Potential in einem Umgebungsbereich der Referenz-Elektrode charakteristisches Signal bereitstellbar ist.

Das beschriebene Verfahren zeichnet sich durch eine besonders einfache Vorgehensweise aus: Alle Substanzen liegen in wässriger Lösung vor, weshalb sie sich für die auch in der Biotechnologie üblichen Druck-Verfahren eignen. Insbesondere sind aufwändige elektrochemische Prozesse (Galvanisieren, Chlorieren) entbehrlich, weshalb das beschriebene Herstellungsverfahren sehr kostengünstig ist. Der Silber-Spiegel 311 weist eine ausreichend gute mechanische Stabilität auf. Insbesondere eignet sich die Schaltkreis-Anordnung 320 dafür, gemeinsam mit einem Biosensor betrieben zu werden, der vorzugsweise auch auf bzw. in dem Substrat 301 integriert sein kann.

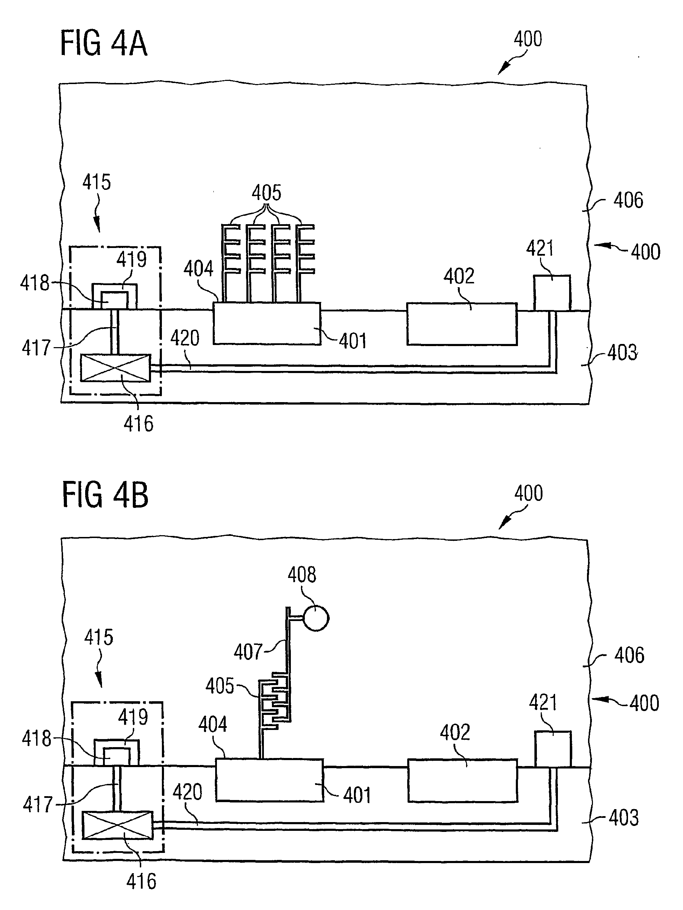

Im Weiteren wird bezugnehmend auf Fig.4A bis Fig.4C eine als Biosensor-Anordnung 400 ausgestaltete Schaltkreis-Anordnung gemäß einem bevorzugten Ausführungsbeispiel der Erfindung beschrieben.

Gemäß der Biosensor-Anordnung 400 können makromolekulare Biomoleküle unter Verwendung eines Reduktions-Oxidations-Recycling-Verfahrens erfasst werden, wobei eine erfindungsgemäße Referenz-Elektrode einen Analyten auf einem konstanten und definierten elektrischen Potential hält.

In **Fig.4A** ist die Biosensor-Anordnung 400 mit einer ersten Arbeits-Elektrode 401 und einer zweiten Arbeits-Elektrode 402 gezeigt, die in einem Silizium-Substrat 403 integriert sind. Auf der ersten Arbeits-Elektrode 401 ist ein Haltebereich 404 aus Gold-Material aufgebracht. Der Haltebereich 404 dient zum Immobilisieren von DNA-Sondenmolekülen 405 auf der ersten Arbeits-Elektrode 401. Auf der zweiten Arbeits-Elektrode 402 ist ein solcher Haltebereich nicht vorgesehen.

Sollen mittels der Biosensor-Anordnung 400 DNA-Stränge 407 mit einer Basensequenz, die komplementär ist zu der Sequenz der immobilisierten DNA-Sondenmoleküle 405, erfasst werden, so wird die Biosensor-Anordnung 400 mit einer zu untersuchenden Lösung, nämlich einem Elektrolyt 406 in Kontakt gebracht, derart, dass in der zu untersuchenden Lösung 406 möglicherweise enthaltene DNA-Stränge 407 mit einer zu der Sequenz der DNA-Sondenmoleküle 405 komplementären Sequenz hybridisieren können.

Um die Biosensor-Anordnung 400 bei definierten Bedingungen betreiben zu können, insbesondere um das elektrische Potential des Elektrolyten 406 konstant zu halten, ist eine integrierte Referenz-Elektroden-Anordnung 415 in dem Silizium-Substrat 403 integriert. Die integrierte Referenz-Elektroden-Anordnung 415 weist einen in dem Silizium-Substrat 403 integrierten Schaltkreis 416 auf. Ferner hat die integrierte Referenz-Elektroden-Anordnung 415 eine auf dem Silizium-Substrat 403 ausgebildete Referenz-Elektrode aus einem Silber-Kern 418, der teilweise von einer Silberchlorid-Umhüllung 419 aus schwerlöslichem Silberchlorid-Salz umgeben ist. Der integrierte Schaltkreis 416 ist mit dem Silber-Kern 418 über ein elektrisch leitfähiges Wolfram-Kopplungsmittel 417 elektrisch gekoppelt. Der integrierte Schaltkreis 416 ist derart eingerichtet, dass ihm von der Referenz-Elektrode ein für das elektrische Potential des Elektrolyten 406 charakteristisches Signal bereitgestellt wird. Ferner ist der Schaltkreis 416 über ein anderes Wolfram-Kopplungsmittel 420 mit einer auf dem Silizium-Substrat 403 angeordneten Gegenelektrode 421 gekoppelt. Der Schaltkreis 416 ist ferner derart eingerichtet, dass er der in den Elektrolyten 406 eintauchenden Gegenelektrode 421 basierend auf dem erfassten Potential ein Steuer-Signal bereitstellt. Dieses ist derart gewählt, dass das elektrische Potential des Elektrolyten 406 mittels der Gegenelektrode 421 derart beeinflusst wird, dass das elektrische Potential des Elektrolyten 406 gleich einem Referenz-Wert ist.

In **Fig.4B** ist ein Szenario gezeigt, gemäß dem in der zu untersuchenden Lösung 406 zu erfassende DNA-Stränge 407 enthalten sind, von denen einer mit einem DNA-Sondenmolekül 405 hybridisiert ist. Die DNA-Stränge 407 in der zu untersuchenden Lösung sind mit einem Enzym 408 markiert, mit dem es möglich ist, im Weiteren beschriebene Moleküle in elektrochemisch aktivierte Teilmoleküle zu spalten. Üblicherweise ist eine erheblich größere Anzahl von DNA-Sondenmolekülen 405 bereitgestellt, als zu ermittelnde DNA-Stränge 407 in der zu untersuchenden Lösung 406 enthalten sind.

Nachdem die in der zu untersuchenden Lösung 406 enthaltenen DNA-Stränge 407 samt dem Enzym 408 mit den immobilisierten DNA-Sondenmolekülen 405 hybridisiert sind, erfolgt vorzugsweise eine spezielle Spülung der Biosensor-Anordnung 400, wobei die hierfür verwendete Spüllösung derart eingerichtet ist, dass solche DNA-Stränge, an denen ein Hybridisierungsereignis nicht stattgefunden hat, entfernt werden, und wodurch die Biosensor-Anordnung 400 von der zu untersuchenden Lösung 406 gereinigt wird. Der zum Spülen verwendeten Spüllösung wird eine elektrochemisch inaktive Substanz beigegeben, die Moleküle enthält, die mittels des Enzyms 408 gespalten werden können in zwei Teilmoleküle 410, von denen mindestens eines elektrochemisch aktiv ist und üblicherweise eine elektrische Ladung aufweist..

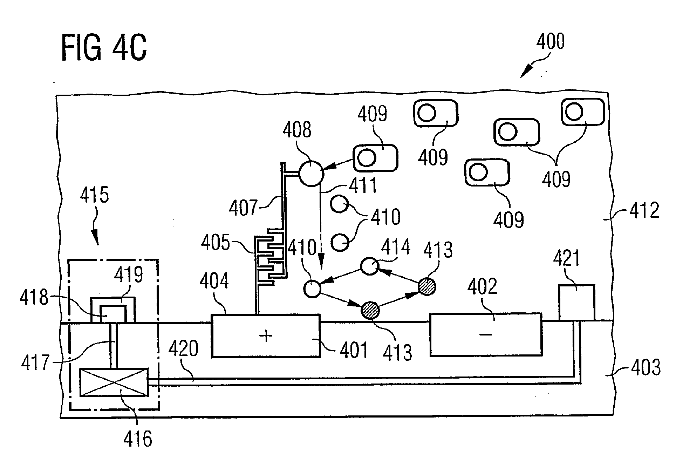

Die gemäß dem beschriebenen Ausführungsbeispiel negativ geladenen Teilmoleküle 410 werden, wie in **Fig. 4C** gezeigt, zu der positiv geladenen ersten Arbeits-Elektrode 201 gezogen, was mittels eines Pfeils 411 in Fig.4C angedeutet ist. Die negativ geladenen Teilmoleküle 410 werden an der ersten Arbeits-Elektrode 401, die ein positives elektrisches Potential aufweist, oxidiert und werden als oxidierte Teilmoleküle 413 an die negativ geladene zweite Arbeits-Elektrode 402 gezogen, wo sie wiederum reduziert werden. Die reduzierten Teilmoleküle 414 wandern wiederum zu der positiv geladenen Arbeits-Elektrode 401. Auf diese Weise wird ein elektrischer Kreisstrom generiert, der proportional ist zu der Anzahl der jeweils mittels der Enzyme 408 erzeugten Ladungsträger.

Während des Betreibens der Biosensor-Anordnung 400 sollen die Oxidations- bzw. Reduktionspotentiale kontrolliert werden. Hierfür ist erforderlich, ein bekanntes elektrisches Potential anzulegen. Anschaulich erfolgt dies mittels der von der Referenz-Elektrode, der Gegenelektrode 421 (und den Arbeits-Elektroden 401, 402) gebildeten Potentiostat-Einrichtung.

Wie aus der obigen Beschreibung hervorgeht, ist die Funktionalität der erfindungsgemäßen Referenz-Elektroden-Anordnung 415 für die Funktionalität der Biosensor-Anordnung 400 essentiell.

In diesem Dokument sind folgende Veröffentlichungen zitiert:

[1] Homepage der Firma Inventus Biotec, http://inventus-biotec.com/deu/produkt/t-sensorchip.html (Stand 9. April 2001)

[2] Homepage der Firma World Precision Instruments, "Dri-Ref™ Referenz-Elektroden", http://www.wpiinc.com/WPI_Web/Biosensing/Dri-Ref.html (Stand 9. April 2001)

[3] Mace, ML, Montagu, J, Rose, SD, McGuinness, G "Novel Microarray Printing and Detection Technology" in: Schena, M (ed.) "Microarray Biochip Technology" (Kapitel 3), Biotechnique Books, Eaton Publishing, ISBN 1881299376

[4] http://pubs.acs.org/subscribe/journals/mdd/v04/i05/html/05zubritsky_table1.html (Stand 9. April 2001)

[5] DE 196 21 996 C2

[6] DE 196 21 997 C1

[7] US 5,672,257

[8] EP 0 588 153 B1

[9] US 5,801,428

Bezugszeichenliste

| | |
|---|---|
| 100 | elektrochemische Experimentier-Anordnung |
| 101 | Behälter |
| 102 | Analyt |
| 103 | erste Arbeits-Elektrode |
| 104 | zweite Arbeits-Elektrode |
| 105 | Stromversorgung |
| 106 | Amperemeter |
| 107 | Voltmeter |
| 108 | Kapillare |
| 109 | Silber/Silberchlorid-Elektroden-Anordnung |
| 110 | Silberdraht |
| 200 | Sensor-Anordnung |
| 201 | Arbeits-Elektrode |
| 202 | Gegen-Elektrode |
| 203 | Referenz-Elektrode |
| 204 | Kontaktierung |
| 300 | Schichtenfolge |
| 301 | Silizium-Substrat |
| 302 | integrierter Schaltkreis |
| 303 | Wolfram-Kontaktierungselement |
| 304 | Gold-Grundelektrode |
| 310 | Schichtenfolge |
| 311 | Silber-Material |
| 320 | Schaltkreis-Anordnung |
| 321 | Silberchlorid-Umhüllung |
| 400 | Biosensor-Anordnung |
| 401 | erste Arbeits-Elektrode |
| 402 | zweite Arbeits-Elektrode |
| 403 | Silizium-Substrat |
| 404 | Haltebereich |
| 405 | DNA-Sondenmolekül |
| 406 | Elektrolyt |
| 407 | DNA-Strang |
| 408 | Enzym |
| 409 | spaltbares Molekül |
| 410 | Teilmoleküle |
| 411 | Pfeil |
| 412 | weitere Lösung |
| 413 | oxidiertes erstes Teilmolekül |
| 414 | reduziertes erstes Teilmolekül |
| 415 | integrierte Referenz-Elektroden-Anordnung |
| 416 | Schaltkreis |
| 417 | Wolfram-Kopplungsmittel |
| 418 | Silber-Kern |
| 419 | Silberchlorid-Umhüllung |

(fortgesetzt)

| 420 | anderes Wolfram-Kopplungsmittel |
| 421 | Gegenelektrode |

**Patentansprüche**

1. Verfahren zum Herstellen einer Biosensor-Schaltkreis-Anordnung,

   • bei dem ein integrierter Schaltkreis in einem Substrat ausgebildet wird;
   • bei dem ein Kern einer integrierten Referenz-Elektrode mittels Bedruckens des Substrats mit Silber-Material als Metall ausgebildet wird;
   • bei dem biologische Moleküle mittels Bedruckens auf Sensor-felder der Biosensor-Schaltkreisanordnung aufgebracht werden, womit die Sensorfelder biologisch aktiviert werden;
   • bei dem das Bedrucken des Substrats mit Silber-Material und das Bedrucken der Sensorfelder mit den biologischen Molekülen in dem gleichen Arbeitsschritt erfolgt;
   • bei dem nachfolgend der Kern aus Silber-Material zumindest teilweise von einer Hülle aus einem schwer löslichen Salz des Silber-Materials umgeben wird, womit die integrierte Referenz-Elektrode ausgebildet wird;
   • bei dem der integrierte Schaltkreis mit dem Kern der integrierten Referenz-Elektrode elektrisch gekoppelt wird.

2. Verfahren zum Herstellen einer Biosensor-Schaltkreis-Anordnung,

   • bei dem ein integrierter Schaltkreis in einem Substrat ausgebildet wird;
   • bei dem ein Kern einer integrierten Referenz-Elektrode aus Silber als Metall ausgebildet wird, indem

      o das Substrat mit Silbersalz-Material bedruckt wird;
      o das Silbersalz-Material chemisch zu Silber reduziert wird;

   • bei dem biologische Moleküle mittels Bedruckens auf Sensor-felder der Biosensor-Schaltkreis-Anordnung aufgebracht werden, womit die Sensorfelder biologisch aktiviert werden;
   • bei dem das Bedrucken des Substrats mit dem Kern der integrierten Referenz-Elektrode und das Bedrucken der Sensorfel-• der mit den biologischen Molekülen und in dem gleichen Arbeitsschritt erfolgt;
   • bei dem nachfolgend der Kern der integrierten Referenz-Elektrode zumindest teilweise von einer Hülle aus einem schwer löslichen Salz des Silbers als Metall umgeben wird, womit die integrierte Referenz-Elektrode ausgebildet wird; und
   • bei dem der integrierte Schaltkreis mit dem Kern der integrierten Referenz-Elektrode elektrisch gekoppelt wird.

3. Verfahren nach Anspruch 1 oder 2,
   bei dem eine elektrisch leitfähige Kopplungsstruktur derart ausgebildet wird, dass mit dieser der integrierte Schaltkreis mit dem Kern elektrisch gekoppelt wird.

4. Verfahren nach Anspruch 3,
   bei dem die Kopplungsstruktur auf und/oder in dem Substrat angeordnet wird, dass der Kern mittels Bedeckens der Kopplungsstruktur und/oder des Substrats mit dem Silber-Material oder dem Silbersalz-Material ausgebildet wird.

5. Verfahren nach einem der Ansprüche 1 bis 4,
   bei dem als Salz des Metalls Silberchlorid verwendet wird.

6. Verfahren nach einem der Ansprüche 1 bis 5, ,
   bei dem der Kern zumindest teilweise von der Hülle umgeben wird, indem der Kern aus Silber unter Verwendung

   • eines elektrochemischen Verfahrens oder
   • eines chemischen Verfahrens

   chloriert wird.

7. Verfahren nach einem der Ansprüche 1 bis 6,

bei welchem der Schaltkreis derart eingerichtet wird, dass ihm von der Referenz-Elektrode ein für das elektrische Potential in einem Umgebungsbereich der Referenz-Elektrode charakteristisches Signal bereitstellbar ist.

8. Verfahren nach einem der Ansprüche 1 bis 7,
   bei dem als Substrat

   • ein Halbleiter-Material;
   • Glas;
   • Kunststoff; und/oder.
   • Keramik

   verwendet wird.

9. Verfahren nach einem der Ansprüche 3 bis 8,
   bei dem die Kopplungsstruktur aus

   • Gold; und/oder
   • Platin

   gebildet wird.


**Claims**

1. A method for producing a biosensor circuit arrangement,

   • in which an integrated circuit is formed in a substrate;
   • in which a core of an integrated reference electrode is formed by means of printing silver material as metal on the substrate;
   • in which biological molecules are applied by means of printing on sensor arrays of the biosensor circuit arrangement, whereby the sensor arrays are biologically activated;
   • in which the printing of silver material on the substrate and the printing of the biological molecules on the sensor arrays are effected in the same work step;
   • in which the core made of silver material is subsequently at least partly surrounded by a sheath made of a sparingly soluble salt of the silver material, thereby forming the integrated reference electrode;
   • in which the integrated circuit is electrically coupled to the core of the integrated reference electrode.

2. A method for producing a biosensor circuit arrangement,

   • in which an integrated circuit is formed in a substrate;
   • in which a core of an integrated reference electrode made of silver as metal is formed by

      o printing silver salt material on the substrate;
      o chemically reducing the silver salt material to form silver;

   • in which biological molecules are applied by means of printing on sensor arrays of the biosensor circuit arrangement, whereby the sensor arrays are biologically activated;
   • in which the printing of the core of the integrated reference electrode on the substrate and the printing of the biological molecules on the sensor arrays are effected and in the same work step;
   • in which the core of the integrated reference electrode is subsequently at least partly surrounded by a sheath made of a sparingly soluble salt of the silver as metal, thereby forming the integrated reference electrode; and
   • in which the integrated circuit is electrically coupled to the core of the integrated reference electrode.

3. The method as claimed in claim 1 or 2,
   in which an electrically conductive coupling structure is formed in such a way that the integrated circuit is electrically coupled to the core by means of said coupling structure.

4. The method as claimed in claim 3,

in which the coupling structure is arranged on and/or in the substrate in such a way that the core is formed by means of covering the coupling structure and/or the substrate with the silver material or the silver salt material.

5. The method as claimed in one of claims 1 to 4,
in which silver chloride is used as the salt of the metal.

6. The method as claimed in one of claims 1 to 5,
in which the core is at least partly surrounded by the sheath by chlorinating the core made of silver using

   • an electrochemical method or
   • a chemical method.

7. The method as claimed in one of claims 1 to 6,
in which the circuit is configured in such a way that a signal characteristic of the electrical potential in a region surrounding the reference electrode can be provided to said circuit by the reference electrode.

8. The method as claimed in one of claims 1 to 7,
in which the substrate used is

   • a semiconductor material;
   • glass;
   • plastic; and/or
   • ceramic.

9. The method as claimed in one of claims 3 to 8,
in which the coupling structure is formed from

   • gold; and/or
   • platinum.

**Revendications**

1. Procédé de fabrication d'un montage biocapteur-circuit

   • dans lequel on forme un circuit intégré dans un substrat ;
   • dans lequel on forme un noyau d'une électrode de référence intégrée au moyen d'une impression du substrat par du matériau d'argent en tant que métal ;
   • dans lequel on dépose des molécules biologiques au moyen d'une impression sur des champs de capteur du montage biocapteur-circuit de sorte que les champs de capteur sont activés biologiquement ;
   • dans lequel on effectue l'impression du substrat par du matériau d'argent et l'impression des champs de capteur par les molécules biologiques dans le même stade opératoire ;
   • dans lequel on entoure ensuite le noyau en matériau d'argent au moins en partie d'une enveloppe en un sel peu soluble du matériau d'argent, en formant ainsi l'électrode de référence intégrée ;
   • dans lequel on couple électriquement le circuit intégré au noyau de l'électrode de référence intégré.

2. Procédé de fabrication d'un montage biocapteur-circuit

   • dans lequel on forme un circuit intégré dans un substrat ;
   • dans lequel on forme un noyau d'une électrode de référence intégrée en argent comme métal,

      o en imprimant le substrat par du matériau de sel d'argent ;
      o en réduisant chimiquement le matériau de sel d'argent en argent ;

   • dans lequel on dépose des molécules biologiques au moyen d'une impression sur des champs de capteur du montage biocapteur-circuit de sorte que les champs de capteur sont activés biologiquement ;
   • dans lequel on effectue l'impression du substrat par le noyau de l'électrode de référence intégré et l'impression des champs de capteur par les molécules biologiques et dans le même stade opératoire ;

EP 1 540 323 B1

• dans lequel ensuite on entoure le noyau de l'électrode de référence intégrée au moins en partie d'une enveloppe en un sel d'argent peu soluble en tant que métal en formant ainsi l'électrode de référence intégrée ;
• dans lequel on couple le circuit intégré au noyau de l'électrode de référence intégrée.

3. Procédé suivant la revendication 1 ou 2,
dans lequel on forme une structure de couplage conductrice d'électricité de façon à coupler électriquement par celle-ci le circuit intégré au noyau.

4. Procédé suivant la revendication 3,
dans lequel on met la structure de couplage sur/ou dans le substrat de façon à ce que le noyau soit formé au moyen du recouvrement de la structure de couplage et/ou du substrat par le matériau d'argent ou par le matériau de sel d'argent.

5. Procédé suivant l'une des revendications 1 à 4,
dans lequel on utilise comme sel du métal le chlorure d'argent.

6. Procédé suivant l'une des revendications 1 à 5,
dans lequel on entoure au moins en partie le noyau de l'enveloppe en chlorant le noyau d'argent en utilisant

   • un procédé électrochimique ou
   • un procédé chimique.

7. Procédé suivant l'une des revendications 1 à 6,
dans lequel le circuit est tel qu'il peut lui être mis à disposition par l'électrode de référence un signal caractéristique du potentiel électrique dans une partie environnante de l'électrode de référence.

8. Procédé suivant l'une des revendications 1 à 7,
dans lequel on utilise comme substrat

   • un matériau semi-conducteur ;
   • du verre ;
   • de la matière plastique ; et/ou
   • de la céramique.

9. Procédé suivant l'une des revendications 3 à 8,
dans lequel la structure de couplage est en

   • or ; et/ou
   • platine

**FIG 2** Stand der Technik

**FIG 1** Stand der Technik

FIG 3A

FIG 3B

FIG 3C

# FIG 4A

# FIG 4B

FIG 4C

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9919057 A
- EP 0299778 A2
- DE 19621996 C2
- DE 19621997 C1
- US 5672257 A
- EP 0588153 B1
- US 5801428 A

**In der Beschreibung aufgeführte Nicht-Patentliteratur**

- Novel Microarray Printing and Detection Technology. **MACE, ML ; MONTAGU, J ; ROSE, SD ; MCGUIN-NESS, G.** Microarray Biochip Technology. Biotechnique Books, Eaton Publishing